# EUROPEAN PATENT SPECIFICATION

(11) **EP 2 358 313 B1**
(45) Date of publication and mention of the grant of the patent: **16.12.2015**
(21) Application number: 09789849.8
(22) Date of filing: 18.06.2009
(51) Int. Cl.: A61F 5/44

(54) **OSTOMY POUCH**
STOMABEUTEL
POCHE DE STOMIE

(30) Priority: 15.09.2008 US 97007 P
(43) Date of publication of application: 24.08.2011
(73) Proprietor: Hollister Incorporated, Libertyville, IL 60048 (US)
(72) Inventor: WHEATON, Amy, Lindenhurst IL 60046 (US); NOWAK, George, McHenry IL 60051 (US)
(74) Representative: Høiberg A/S
(86) International application number: PCT/US2009/047830
(87) International publication number: WO 2010/030426

(56) References cited:
- WO-A-93/17643
- WO-A-2004/100842
- DE-U1- 8 709 793
- US-A- 2 741 247
- US-A1- 2007 262 479

## Description

### Field of the Disclosure

The present disclosure is generally directed to a pouch which is suitable for the collection of a liquid or semisolid body waste material and, more particularly, to an ostomy pouch which is adapted to receive such body waste material through a stomal opening for later disposal. An ostomy pouch of this kind and as described in the preamble of claim 1 is known from WO 93/17643.

### Background of the Disclosure

Ostomy pouches for the collection of liquid or semisolid body waste material are well known and typically include flat, opposing side walls secured together along their edges to define a human stomal discharge collection cavity. One of the side walls is provided with an opening to receive a stoma, and means such as a connecting flange is provided for securing the pouch to an adhesive barrier placed to surround the stoma so that body waste material discharged through the stoma will be received within the cavity. At its lower end, the ostomy pouch may have a discharge opening which may be closed during collection of the liquid or semisolid body waste material that passes through the stoma but may be opened for draining the body waste material from the pouch after a period of use. Alternatively, the ostomy pouch may be designed for a single use in which case it will not be provided with a discharge opening since the entire pouch will be discarded after it has substantially filled with human stomal discharge.

A drainable pouch is typically reusable following periodic emptying of the body waste material by utilizing a closure for the discharge opening that may take a number of different forms so long as it serves to prevent leakage of the body waste material whereas there is no need for a closure in the case of a single use pouch since it is intended to be discarded following use.

In either case, it is known that ostomy pouches often show bulkiness after a period of time of accumulating body waste material. This is true with respect to both liquid and semisolid body waste material because the normally flat side walls of such pouches are formed of a very thin film which easily expands outwardly. It is also known that ostomy pouches are sized to accommodate a certain period of use which makes it difficult to conceal them beneath clothing even before they fill with body waste material. However, after ostomy pouches do fill with human stomal discharge, they can expand outward significantly to create an obvious bulge.

While the foregoing is true for both drainable ostomy pouches and single use ostomy pouches, there is still an additional important consideration. A manufacturer of ostomy products commonly has a high number of SKUs devoted to its ostomy product portfolio due to the differing requirements in terms of size and capacity for those who need to use ostomy pouches for collection of human stomal discharge. If the number of SKUs could be reduced, it would be of great benefit to both the manufacturers and the users of ostomy pouches.

In particular, a reduction in the number of SKUs would reduce the cost of manufacturing, marketing and selling ostomy pouches which would result in a reduction of the cost of ostomy pouches to the user. As a result, there has been a need to address the desire to have ostomy pouches that are more easily concealed beneath clothing as well as the desire to reduce the number of SKUs for ostomy pouches.

### Summary of the Disclosure

The present disclosure is directed to an ostomy pouch according to claim 1. The pouch comprises proximal and distal side walls of flexible sheet material joined together along their peripheral edges to define a cavity therebetween. The side walls are also joined in at least one location inwardly of the peripheral edges to limit separation of the proximal and distal side walls. With this construction, the side walls also include a laterally outwardly expandable fold which is formed by at least a portion of at least one of the side walls.

The peripheral edges of the proximal and distal side walls are joined together by welding. Similarly, the side walls are joined at the at least one location inwardly of the peripheral edges by welding.

The proximal and distal side walls may be joined by welding at a plurality of locations inwardly of the peripheral edges in order to thereby limit the separation of the proximal and distal side walls.

In yet another embodiment, the laterally outwardly expandable fold is formed by substantially the entirety of the joined peripheral edges. Further, the ostomy pouch may be formed to have a top edge, a bottom edge and opposite side edges extending between the top edge and the bottom edge. In this case, a laterally outwardly expandable fold may suitably be formed along each of the opposite side edges of the ostomy pouch.

In still another embodiment, the ostomy pouch may be generally rectangular in shape and the opposite sides may be generally parallel such that the laterally outwardly expandable folds are generally parallel. With a generally rectangular shaped ostomy pouch, it may also include a generally parallel top and bottom in which case a laterally outwardly expandable fold may be formed in at least the bottom of the pouch.

According to the invention, the laterally outwardly expandable folds are each secured by an adhesive spot weld which is subject to degradation from exposure to human stomal discharge in the cavity of the ostomy pouch.

Other advantages and features of the present disclosure will become apparent from the following specification taken in conjunction with the accompanying drawings.

### Brief Description of the Drawings

Figure 1 is a front elevational view of a first ostomy pouch ;
Figure 2 is a cross-sectional view taken along the line 2-2 of Figure 1 illustrating the side walls joined together at a point inwardly of the peripheral edges;
Figure 3 is a view similar to Figure 2 illustrating a fold expanded laterally outwardly to accommodate an additional volume of human stomal discharge;
Figures 4A-4C are cross-sectional views illustrating various additional laterally outwardly expandable folds before and after expansion thereof;
Figure 5 is a front elevational view of a second ostomy pouch ;
Figure 6 is a cross-sectional view taken along the line 6-6 of Figure 5 illustrating a laterally outwardly expandable fold before expansion thereof;
Figure 7 is a front elevational view of Figure 5 with folds expanded laterally outwardly to accommodate an additional volume of human stomal discharge;
Figure 8 is a cross-sectional view taken along the line 8-8 of Figure 7 illustrating a laterally outwardly expandable fold after expansion thereof; and
Figure 9 is a front elevational view of an ostomy pouch in accordance with the present invention.

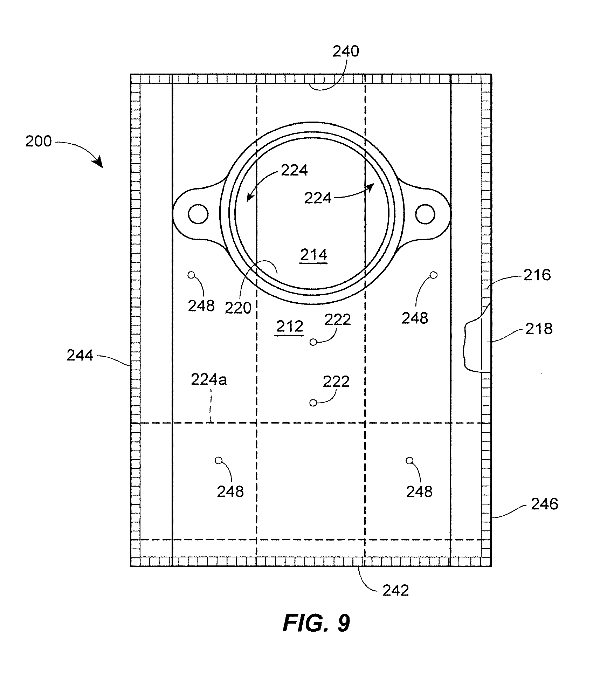

Only Fig. 9 reflects an ostomy pouch as defined in claim 1.

### Detailed Description of the Present Disclosure

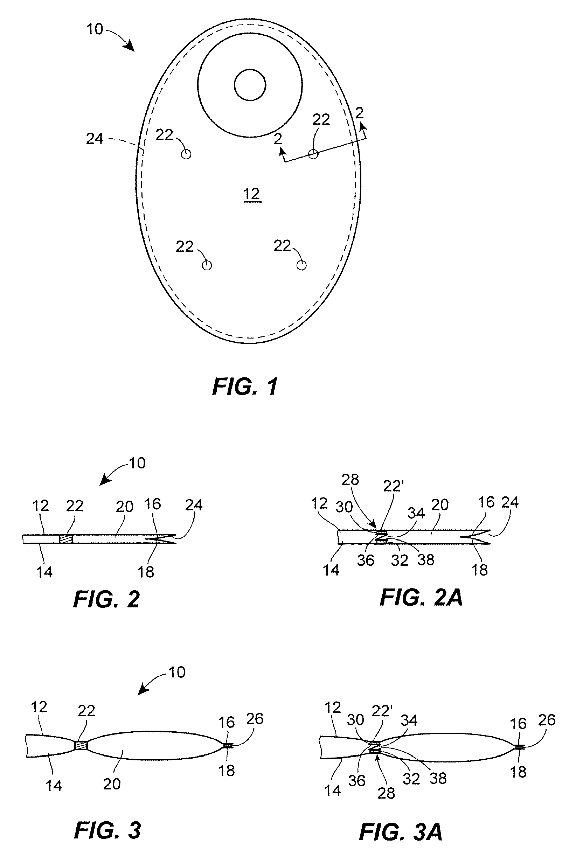

In the various illustrations given, and with reference first to Figures 1-3, a first embodiment of an ostomy pouch according to the present disclosure is generally designated by the reference 10. The ostomy pouch 10 is formed of a proximal side wall 12 and a distal side wall 14 of flexible sheet material which are joined together along their peripheral edges 16 and 18, respectively, to define a cavity 20 for receiving human stomal discharge. In addition, the side walls 12 and 14 are also joined in at least one location such as 22 inwardly of the peripheral edges 16 and 18 to thereby limit the separation of the proximal and distal side walls 12 and 14.

Referring to Figures 2 and 3, it will be seen that the side walls 12 and 14 include a laterally outwardly expandable fold 24 formed by at least a portion of the joined peripheral edges 16 and 18 thereof. The peripheral edges 16 and 18 of the proximal and distal side walls 12 and 14 may be joined together by welding as at 26 as best shown in Figure 3. As shown in Figures 2 and 3, the proximal and distal side walls 12 and 14 also may be joined together by welding or the like as at 22 to thereby limit the extent to which they may separate from one another.

As an alternative illustrated in Figures 2A and 3A, the proximal and distal side walls 12 and 14 may be joined together at one or more locations such as 22' inwardly of the peripheral edges 16 and 18 by a baffle 28. The baffle 28 may be z-shaped and secured to the proximal and distal side walls 12 and 14 as at 30 and 32 to limit separation thereof. In particular, the baffle 28 may have a central web 34 extending between a proximal end portion 36 welded to the proximal side wall 12 as at 30 and a distal end portion 38 welded to the distal sidewall 14 as at 32 .

Regardless of whether the proximal and distal side walls 12 and 14 are joined together by welding, adhesive, or baffles, it may be desirable depending upon the size of the ostomy pouch 10 for the proximal and distal side walls 12 and 14 to be joined together at a plurality of locations such as 22 inwardly of the peripheral edges 16 and 18 as shown in Figure 1.

Still referring to Figure 1, it will be seen that the laterally outwardly expandable fold 24 is formed by substantially the entirety of the peripheral edges 16 and 18 of the proximal and distal side walls 12 and 14 of the ostomy pouch 10. By providing the laterally outwardly expandable fold 24 entirely about the perimeter of the ostomy pouch 10, it will be appreciated that it is possible to manufacture the pouch in a smaller size than conventional pouches while not reducing the overall capacity of the cavity 20 to hold human stomal discharge since the cavity 20 can expand to a considerable degree. Specifically, the laterally outwardly expandable fold 24 can expand entirely about the perimeter of the ostomy pouch 10 and, depending upon the form of the fold 24, the additional capacity of the cavity 20 can be increased significantly.

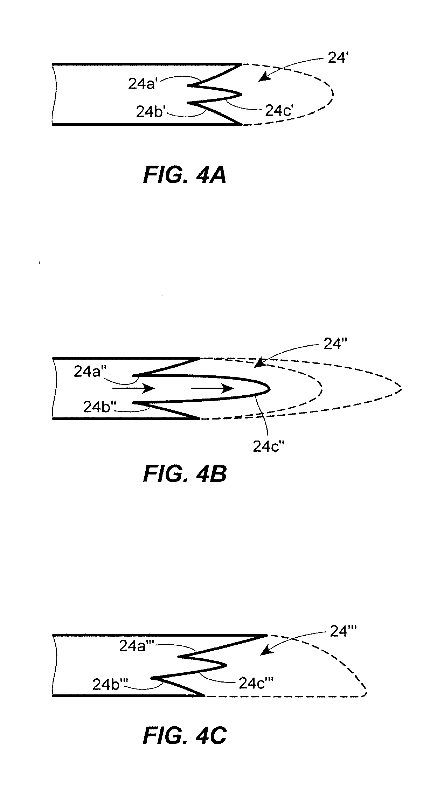

Referring to Figures 2 and 3, it will be seen that the laterally outwardly expandable fold 24 is comprised of a single inwardly directed fold wherein the outermost extremes of the proximal and distal side walls 12 and 14 where the peripheral edges 16 and 18 are welded as at 26 initially point inwardly of the cavity 20. However, it is also possible to form the laterally outwardly expandable fold so it comprises a double fold such as the folds 24', 24" and 24'" which are shown in Figures 4A, 4B and 4C. As will be appreciated, the double folds 24', 24" and 24'" each include a pair of inwardly directed folds 24a' and 24b', 24a" and 24b", and 24a"' and 24b'" as well as a single outwardly directed fold 24c', 24c" and 24c"' which points outwardly of the cavity 20 both before and after the fold expands outwardly.

To understand the laterally outwardly expandable folds 24', 24" and 24"', the folds have been shown in Figures 4A, 4B and 4C in solid lines where the cavity 20 as an initial capacity for receiving human stomal discharge. The folds 24', 24" and 24'" have also been shown in Figures 4A, 4B and 4C in dashed lines where the cavity 20 has a maximum capacity for receiving human stomal discharge after the ostomy pouch 10 has been used for a time during which the cavity 20 has filled beyond its initial capacity. By utilizing any one of the double folds 24', 24" and 24"', it is possible to enhance the maximum capacity of the cavity 20 receiving human stomal discharge beyond what can be achieved with the single fold 24.

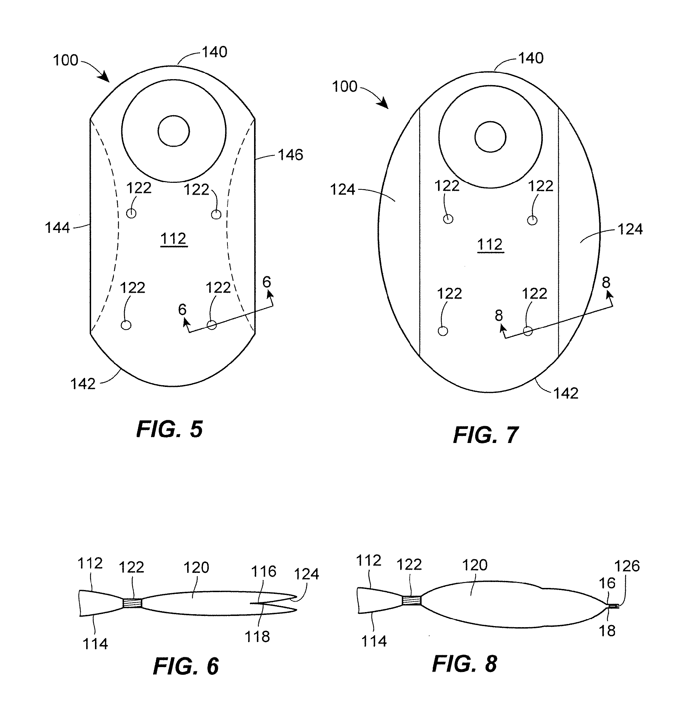

Referring now to Figures 5-8, a second embodiment of an ostomy pouch according to the present disclosure generally designated by the reference 100 is formed of a proximal side wall 112 and a distal side wall 114 of flexible sheet material. The side walls 112 and 114 are joined together as by welding or the like along their peripheral edges 116 and 118, respectively, to define a cavity 120 for receiving human stomal discharge and, in this embodiment, the side walls 112 and 114 are shaped such that the ostomy pouch 100 has a top edge 140, a bottom edge 142, and a pair of opposite side edges 144 and 146. Also, the side walls 112 and 114 are joined in one or more locations such as 122 inwardly of the peripheral edges 116 and 118 to thereby limit the amount by which the proximal and distal side walls 112 and 114 may separate from one another.

Referring to Figures 5 and 7, it will be seen that the side walls 112 and 114 include a laterally outwardly expandable fold 124 formed along each of the opposite side edges 144 and 146 of the ostomy pouch 100. The peripheral edges 116 and 118 of the side walls 112 and 114 may be joined together by welding as at 126 as best shown in Figure 8. As shown in Figures 6 and 8, the proximal and distal side walls 112 and 114 may also be joined together by welding or the like as at 122 to thereby limit the extent to which they may separate from one another.

Referring now to Figure 9, an ostomy pouch according to the present invention generally designated by the reference numeral 200 is formed of a proximal side wall 212 and a distal side wall 214 of flexible sheet material. The side walls 212 and 214 are joined together by welding along peripheral edges 216 and 218, respectively, to define a cavity 220 for receiving human stomal discharge and, in this embodiment, the side walls 212 and 214 are generally rectangular so the ostomy pouch 200 has a top edge 240, a bottom edge 242, and parallel side edges 244 and 246. Also, the side walls 212 and 214 are joined in one or more locations such as 222 inwardly of the peripheral edges 216 and 218 to thereby limit the amount by which the proximal and distal side walls 212 and 214 may separate from one another.

As shown in Figure 9, it will be seen that the side walls 212 and 214 include laterally outwardly expandable folds 224 which are formed by inward folding of the side wall 212 of the ostomy pouch 200. The peripheral edges 216 and 218 of the side walls 212 and 214 are joined together by welding as was described for the other embodiments. As also shown in Figure 9, the ostomy pouch 200 may have not only the outwardly expandable folds 224, but also at least one additional outwardly expandable fold 224a along the bottom edge 242.

In addition, the laterally outwardly expandable folds 224 and 224a are secured by one or more adhesive spot welds as at 248 which are subject to degradation from exposure to human stomal discharge in the cavity 220.

While in the foregoing there have been set forth representative embodiments of the present disclosure, it will be appreciated that the details herein given may be varied by those skilled in the art without departing from the scope of the appended claims. For example, a single piece of flexible sheet material may be used to form the side walls of an ostomy pouch, or alternatively, two or more separate pieces of flexible sheet material may be used to form the side walls of an ostomy pouch, in accordance with the disclosure.

## Claims

1. An ostomy pouch, comprising:
a pair of side walls (212, 214) of flexible sheet material joined together by welding along their peripheral edges to define a cavity therebetween, the side walls (212, 214) being joined by welding in a plurality of spots inwardly of the peripheral edges (216, 218) to limit separation of the side walls as the cavity fills with human stomal discharge, the pouch including a pair of opposite sides each including a laterally outwardly expandable fold (224) formed by at least a portion of at least one of the side walls (212, 214), the folds (224) expanding laterally outwardly as the cavity fills with human stomal discharge to accommodate an increasing volume, whereby the increasing volume of human stomal discharge is accommodated while at the same time maintaining a limit on separation of the side walls (212, 214) during use of the pouch, **characterized in that** the laterally outwardly expandable folds (224) are each secured by an adhesive spot weld (248) subject to degradation from exposure to human stomal discharge in the cavity.

2. The ostomy pouch of claim 1 wherein the pouch is generally rectangular in shape and the opposite sides (240, 242; 244, 246) are generally parallel such that the laterally outwardly expandable folds (224) are generally parallel.

3. The ostomy pouch of claim 1 wherein the pouch is generally rectangular in shape and includes a generally parallel top and bottom (240, 242) with a laterally outwardly expandable fold (224) in at least the bottom (242).

## Patentansprüche

1. Stomabeutel aufweisend:
ein Paar von Seitenwänden (212, 214) aus flexiblem Folienmaterial, die durch Schweißen entlang ihrer Umfangsränder derart miteinander verbunden sind, dass sie einen Hohlraum dazwischen definieren, wobei die Seitenwände (212, 214) durch Schweißen an einer Vielzahl von Stellen von den Umfangsrändern (216, 218) nach innen derart verbunden sind, dass die Trennung der Seitenwände, wenn sich der Hohlraum mit menschlicher Stoma-Ausscheidung füllt, begrenzt ist, wobei der Beutel ein Paar von gegenüberliegenden Seiten aufweist, die jeweils eine seitlich nach außen spreizbare Faltung (224) aufweisen, die durch mindestens einen Abschnitt von mindestens einer der Seitenwände (212, 214) gebildet wird, wobei sich die Faltung (224) seitlich nach außen spreizt, wenn sich der Hohlraum mit menschlicher Stoma-Ausscheidung füllt, um ein ansteigendes Volumen aufzunehmen, wodurch das ansteigende Volumen an menschlicher Stoma-Ausscheidung aufgenommen wird während gleichzeitig eine Begrenzung der Trennung der Seitenwände (212, 214) während der Verwendung des Beutels beibehalten wird, **dadurch gekennzeichnet, dass** die seitlich nach außen spreizbaren Faltungen (224) jeweils durch eine klebende Punktschweißung (248) fixiert sind, die durch den Kontakt mit menschlicher Stoma-Ausscheidung eine Schwächung erfährt.

2. Stomabeutel nach Anspruch 1, wobei der Beutel im Wesentlichen rechteckförmig ist und die gegenüberliegenden Seiten (240, 242; 244, 246) im Wesentlichen parallel sind, so dass die seitlich nach außen spreizbaren Faltungen (224) im Wesentlichen parallel sind.

3. Stomabeutel nach Anspruch 1, wobei der Beutel im Wesentlichen rechteckförmig ist und im Wesentlichen parallele Ober- und Unterseiten (240, 242) mit einer seitlich nach außen spreizbaren Faltung (224) in mindestens der Unterseite (242) aufweist.

## Revendications

1. Poche de stomie, comprenant :
une paire de parois latérales (212, 214) en un matériau de feuille flexible jointes l'une à l'autre par soudure le long de leurs bords périphériques pour définir une cavité entre celles-ci, les parois latérales (212, 214) étant jointes par soudure en une pluralité de points à l'intérieur des bords périphériques (216, 218) pour limiter la séparation des parois latérales alors que la cavité se remplie d'évacuation stomale humaine, la poche comprenant une paire de côtés opposés comprenant chacun un pli déployable latéralement vers l'extérieur (224) formé par au moins une partie d'au moins l'une des parois latérales (212, 214), les plis (224) se déployant latéralement vers l'extérieur alors que la cavité se remplit d'évacuation stomale humaine pour recevoir un volume croissant, le volume croissant de décharge stomale humaine étant ainsi reçu tout en maintenant en même temps une limite à la séparation des parois latérales (212, 214) durant l'utilisation de la poche, **caractérisée en ce que** chacun des plis déployables latéralement vers l'extérieur (224) est fixé par soudure adhésive par points (248) soumise à dégradation due à l'exposition à l'évacuation stomale humaine dans la cavité.

2. Poche de stomie selon la revendication 1, dans laquelle la poche est généralement de forme rectangulaire et les côtés opposés (240, 242 ; 244, 246) sont généralement parallèles de telle sorte que les plis déployables latéralement vers l'extérieur (224) sont généralement parallèles.

3. Poche de stomie selon la revendication 1, dans laquelle la poche est généralement de forme rectangulaire et comprend une partie supérieure et une partie inférieure généralement parallèles (240, 242) avec un pli déployable latéralement vers l'extérieur (224) au moins dans la partie inférieure (242).
